(19) Europäisches Patentamt
European Patent Office
Office européen des brevets

(11) Publication number: 0 314 663
A1

(12) # EUROPEAN PATENT APPLICATION

(21) Application number: 88870164.6

(51) Int. Cl.4: C 07 D 277/80

(22) Date of filing: 28.10.88

(30) Priority: 29.10.87 GB 8725334

(43) Date of publication of application:
03.05.89 Bulletin 89/18

(84) Designated Contracting States:
BE DE ES FR GB IT NL

(71) Applicant: MONSANTO EUROPE S.A.
Avenue de Tervuren 270-272 Letter Box 1
B-1150 Brussels (BE)

(72) Inventor: Peemans, Rudy F. A. J.
Biststraat 55
B-3092 Nederokkerzeel (BE)

Delbrassinne, Pascal G. J.
Rue de la Grande Bruyère 25
B-1330 Rixensart (BE)

Cobb, Alec S.
J. Kumpsstraat 130
B-1990 Hoeilaart (BE)

(74) Representative: Lunt, John Cooper et al
Monsanto Europe S.A. Patent Department Avenue de Tervuren 270-272 Letter Box No 1
B-1150 Brussels (BE)

(54) **Process for the preparation of benzothiazole-2-sulphenamides.**

(57) In a process for the production of benzothiazole-2-sulphenamides, a 2-mercaptobenzothiazole in the form of a particulate solid or a dispersion of a particulate solid in a liquid carrier is added gradually and concurrently with an aqueous solution of sodium hypochlorite to a solution of an amine in a mixture of water and a water-miscible organic solvent, the amount of amine being not more than 1.25 moles per mole of 2-mercaptobenzothiazole. A feature of the process is that it represents a significant improvement over prior art methods for the production of sulphenamides derived from secondary amines such as dicyclohexylamine and diisopropylamine.

EP 0 314 663 A1

## Description

### PROCESS FOR THE PREPARATION OF BENZOTHIAZOLE-2-SULPHENAMIDES

This invention relates to a process for the production of benzothiazole-2-sulphenamides, more particularly a process for the production of N,N-disubstituted benzothiazole-2-sulphenamides.

Benzothiazole-2-sulphenamides are widely used as accelerators in the vulcanisation of rubber, and have been known for many years. Various processes for their production have been proposed, most of which involve an oxidative condensation of 2-mercaptobenzothiazole, a salt of 2-mercaptobenzothiazole, or the corresponding disulphide, with an amine. Sulphenamides derived from primary amines are generally obtainable in higher yields than those derived from secondary amines. The problem of inefficient utilisation of raw materials is particularly acute with amines where one or both of the groups attached to the nitrogen atom is a cycloalkyl or secondary alkyl group.

BE-A-897 708 describes a process for the production of benzothiazole-2-sulphenamides from secondary amines wherein each group attached to the nitrogen atom of the amine is a cycloalkyl or secondary alkyl group. In this process, an aqueous solution of the sodium, potassium or calcium salt of 2-mercaptobenzothiazole is added concurrently with sodium hypochlorite solution to an aqueous or aqueous/alcoholic solution of the amine. Acid is also added to control the pH of the reaction mixture within the range 9-12.

US-A-4 258 197 describes a process for the preparation of benzothiazole-2-sulphenamides, in which an oxidising agent such as sodium hypochlorite is added slowly to a mixture prepared from 2-mercaptobenzothiazole, amine, and water or water and solvent. The process employs from 1.5 to 4 moles of amine per mole of 2-mercaptobenzothiazole.

The present invention provides an improved process for the production of benzothiazole-2-sulphenamides by the oxidative condensation of a 2-mercaptobenzothiazole with an amine. The process is characterised by adding the 2-mercaptobenzothiazole, in the form of particulate solid as such or as a dispersion of particulate solid in a suitable liquid carrier, and an aqueous solution of sodium hypochlorite concurrently to a solution of the amine in a mixture of water and water-miscible organic solvent, the molar ratio of amine to 2-mercaptobenzothiazole being not greater than 1.25:1.

Compared with the process of BE-A-897 708, the process of the invention has the advantages that it is simpler by virtue of not requiring continuous acid addition. Moreover, the use of the 2-mercaptobenzothiazole in the form of a solid instead of as an aqueous solution of a 2-mercaptobenzothiazole salt allows more product to be produced per unit volume of reactor space, and reduces the volume of effluent per unit weight of product. Yields of product obtained, as percentages of theoretical, are substantially the same; overall, therefore, the process of the invention is more efficient.

Compared with the process of US-A-4 258 197, the process of the invention uses significantly less amine relative to the 2-mercaptobenzothiazole. Since the efficient recovery of excess amine is often quite difficult, the need for less amine in the process initially is a significant advantage. Moreover, it is not apparent from the disclosure in US-A-4 258 197 that the process therein described is applicable to the production of benzothiazole-2-sulphenamides from secondary amines where at least one of the groups attached to the nitrogen atom is cycloalkyl. In contrast, the process of the present invention is particularly useful in such instances.

Other prior art methods that can be used for the production in high yield of sulphenamides from primary amines such as cyclo hexylamine or tert-butylamine have not been successfully adapted for the production of sulphenamides derived from secondary amines.

Amines useful in the present process can be represented by the formula

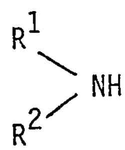

where $R^1$ represents a hydrogen atom or an alkyl or cycloalkyl group and $R^2$ represents an alkyl or cycloalkyl group, or where $R^1$ and $R^2$ together with the nitrogen atom form a ring. Alkyl groups in such amines are generally those containing not more than 10 carbon atoms, and cycloalkyl groups are generally those having 5, 6 or 7 ring carbon atoms. Alkyl groups can be straight-chain or branched; cycloalkyl groups include alkylcycloalkyl groups having a total of up to 9 carbon atoms. Amines with the $>$NH as part of a ring can have, for example 5, 6 or 7 ring members, at least one of which may be another hetero-atom such as oxygen.

Specific examples of alkyl groups are ethyl, n-propyl, isopropyl, sec-butyl, tert-butyl, isopentyl, and 1,1,3,3-tetramethylbutyl. Examples of cycloalkyl groups are cyclopentyl, cyclohexyl and methylcyclohexyl.

The process is particularly useful for the production of sulphenamides derived from secondary amines of the above formula where each of $R^1$ and $R^2$ is independently selected from secondary alkyl groups having up to 10 carbon atoms and cycloalkyl groups having 5, 6 or 7 ring carbon atoms including alkylcycloalkyl groups having a total of up to 9 carbon atoms, or wherein $R^1$ and $R^2$ together with the nitrogen atom form a ring having 5 or 6 ring members, one of which may be oxygen.

Amines that can be used in the process thus include primary amines such as cyclohexylamine, isopropylamine, tert-butylamine and 1,1,3,3-tetramethylbutylamine; secondary amines such as diethylamine, diisopropylamine, cyclohexyl sec-butylamine and dicyclohexylamine; and cyclic amines such as piperidine, morpholine and 2,6-dimethylmorpholine.

Commercially, the important sulphenamides are those derived from 2-mercaptobenzothiazole itself. However, the process of the invention is applicable to the production of sulphenamides from 2-mercaptobenzothiazoles having one or more substituents, which may be, for example, alkyl groups, halogen atoms or nitro groups, on the benzene nucleus.

During the concurrent addition of the sodium hypochlorite solution and the 2-mercaptobenzothiazole, the amount of sodium hypochlorite added per unit time will usually be at least one mole per mole of 2-mercaptobenzothiazole. Preferably the amount is from 1.0 to 1.3 moles, for example from 1.15 to 1.25 moles, of sodium hypochlorite per mole of 2-mercaptobenzothiazole. Commercially available sodium hypochlorite solutions having a concentration of about 12-15% by weight are suitable for use in the process of the invention. To ensure that the conversion of the 2-mercaptobenzothiazole has been carried as far as possible, it is preferred to continue the addition of the sodium hypochlorite after the completion of the addition of the 2-mercaptobenzothiazole. The total amount of sodium hypochlorite added may be, for example, up to 1.5 moles, for example from 1.15 to 1.45 moles, per mole of 2-mercaptobenzothiazole used in the process. Any eventual excess can be eliminated by the addition of sodium sulphite. The pH of the reaction mixture remains substantially constant throughout most of the reaction but increases as the addition of the sodium hypochlorite continues after the total amount of 2-mercaptobenzothiazole has been added. This increase in pH can be correlated with the excess of hypochlorite and can be used to indicate the end point of the addition.

In the process of the invention, the 2-mercaptobenzothiazole and sodium hypochlorite are added to a solution of the amine in a mixture of water and a water-miscible solvent. Preferred organic solvents are monohydric alcohols, for instance an alkanol such as methanol, ethanol, propanol or isopropanol. Other solvents, for example ethylene glycol or tetrahydrofuran can be used, either as such or as a mixture with a monohydric alcohol.

Suitable liquid carriers for use when the 2-mercaptobenzothiazole is added to the reaction system as a dispersion of particulate solid in a liquid carrier, include water, water-miscible organic solvents and mixtures thereof. The water-miscible organic solvent will generally be the same as, or should be compatible with, the water-miscible organic solvent used in the amine solution, examples of which are given above. The function of the liquid carrier is merely to provide the 2-mercaptobenzothiazole in a form, i.e. a dispersion or slurry, which is more fluid than the solid itself. Whether this is necessary depends on the type of equipment available for effecting the addition of the 2-mercaptobenzothiazole to the reaction system. For example a slurry or paste is in general more readily pumped than a particulate solid. The amount of carrier liquid employed will normally be the minimum consistent with obtaining the desired fluidity.

In preferred embodiments of the process, the amounts of water and water-miscible solvent in the solution of the amine are selected so that most or all of the sulphenamide product remains in solution at the temperature at which the reaction is carried out, but crystallises from the solution towards the end of the reaction and on cooling the reaction mixture when the reaction is complete. It will be understood that various factors, including the particular sulphenamide being produced, the organic solvent selected and the temperature at which the reaction is carried out, determine the optimum composition and amount for the mixed solvent. The selection is made with the object of achieving, as far as possible, a single liquid phase throughout the reaction. It may, however, be difficult to avoid some separation of phases as the amount of water increases towards the end of the reaction, especially if a stoichiometric excess of an amine having low solubility in water is present. It has been found that for the production of N,N-dicyclohexylbenzothiazole-2-sulphenamide using isopropanol as the solvent, the preferred weight ratios of isopropanol to water in the initial solution of the amine are from 1:1.3 to 1:1.6, while for the production of N,N-diisopropylbenzothiazole-2-sulphenamide and of 2-(morpholinothio)benzothiazole using isopropanol as the solvent, the preferred weight ratios of isopropanol to water in the initial solution of the amine are from 2.5:1 to 3.5:1 when operating within the optimum temperature range referred to below. Under such conditions, the preferred concentration of dicyclohexylamine in the initial amine solution is from 10 to 15% by weight of the solution, while the preferred concentration of diisopropylamine and of morpholine are within the range 30-45% by weight of the solution.

Reaction temperatures within the range 45° to 80°C have been found to be generally suitable in the operation of the process of the invention, but there is no reason to suppose that somewhat higher or lower temperatures could not be employed. However, the optimum temperature for the production of most sulphenamides is expected to be within the range 45 to 65°C, and is, for exemple, 50°C to 60°C for the production of N,N-dicyclohexylbenzothiazole-2-sulphenamide, 45° to 55°C for the production of N,N-diisopropylbenzothiazole-2-sulphenamide and 55° to 65°C for the production of 2(morpholinothio)benzothiazole.

A feature of the present process is that the amount of amine employed is not more than 1.25 moles per mole of 2-mercaptobenzothiazole, and is preferably not more than 1.1 moles per mole of 2-mercaptobenzothiazole. Under certain conditions, it may be possible to achieve substantially complete conversion of the amine; more usually, however, there is some residual amine when all the 2-mercaptobenzothiazole has been consumed, but its amount and therefore the cost of its recovery are not excessive. The amount of amine employed can be, for example, from 0.8 to 1.05 mole per mole of 2-mercaptobenzothiazole, and for the production of

N,N-dicyclohexylbenzothiazole-2-sulphenamide can be from 0.85 to 0.95 mole per mole of 2-mercaptobenzothiazole.

The invention is illustrated by the following Examples.

Example 1

A stirred mixture of water (244.9 g), isopropanol (169.7 g) and dicyclohexylamine (58.7 g, 0.324 mole) was heated to 55°C in a reaction vessel. 2-Mercaptobenzothiazole (60.2 g, 0.36 mole) in the form of a free-flowing crystalline solid was added to the mixture via a rotary valve at the rate of 0.9 g per minute, concurrently with a solution of 14% by weight sodium hypochlorite added at a rate of 3.45 g per minute. After 60 minutes, the rates of addition were reduced by half. The addition of the sodium hypochlorite solution was continued after the addition of the 2-mercaptobenzothiazole was complete until the pH of the reaction mixture had risen to within the range 10 to 10.5. Throughout the addition of the sodium hypochlorite solution and for 15 minutes after its completion, the temperature of the mixture was held at 55°C. A solution of sodium sulphite (2.9 g) in water (14.4 g) was added, stirring was continued for 5 minutes, and the reactor contents were then cooled to 20°C.

N,N-dicyclohexylbenzothiazole-2-sulphenamide was separated by filtration and washing, firstly with cold (20°C) aqueous isopropanol (179 g) containing 76% by weight of isopropanol and then with water (650 g) at 60°C. The product was dried at 60°C. The amount recovered was 100 g, which represents an 80.3% yield on 2-mercaptobenzothiazole and an 89.2% yield on dicyclohexylamine. The product had an assay by $H_2S$ reduction of 99.0% and a melting point of 102.2°C.

Example 2

A stirred mixture of water (15.0 g), isopropanol (47.1 g) and diisopropylamine (37.2 g, 0.3675 mole) was heated to 50°C in a reaction vessel. 2-mercaptobenzothiazole (58.5 g, 0.35 mole) in the form of a free-flowing crystalline solid was added to the mixture via a rotary valve at the rate of 0.94 g per minute, concurrently with a solution of 14.6% by weight sodium hypochlorite added at a rate of 3.44 g per minute. The addition of sodium hypochlorite continued for 5 minutes after the addition of the 2-mercaptobenzothiazole until the pH of the reaction mixture had risen to 10.0. Throughout the addition of the sodium hypochlorite solution and for 10 minutes after its completion, the temperature of the mixture was held at 50°C. 500 g of ambient water were then added over 10 minutes and the diluted mixture cooled to 10°C.

N,N-diisopropylbenzothiazole-2-sulphenamide was separated by filtration and washing with 500 g of water at ambient temperature. The amount recovered was 78.1 g, which represents an 83.8% yield on the 2-mercaptobenzothiazole used. The melting point of the product was found to be 55.8°C.

Example 3

A stirred mixture of water (17.0 g), isopropanol (45.6 g) and morpholine (46.7 g, 0.537 mole) was heated to 60°C in a reaction vessel. 2-Mercaptobenzothiazole (87.0 g, 0.52 mole) in the form of a free-flowing crystalline solid was added to the mixture via a rotary valve at the rate of 1.38 g per minute concurrently with a solution of 14.6% by weight sodium hypochlorite added at a rate of 4.5 g per minute. The addition of the sodium hypochlorite continued for 9 minutes after the addition of the 2-mercaptobenzothiazole at a rate of 2.25 g per minute until an endpoint indicated by an inflexion in the pH curve. Throughout the addition of the sodium hypochlorite solution and for 10 minutes after its completion, the temperature of the mixture was held at 60°C. A solution of sodium sulphite (1.2 g) in water (50.0 g) was added, and stirring was continued for 10 minutes. A solution of sodium hydroxide (0.6 g) in water (2.5 g) was added, stirring was continued for a further 10 minutes, and the reactor contents were then cooled to 20°C.

2-(morpholinothio)benzothiazole was separated by filtration and washing with water (500 g) at 50°C. The product was dried at 60°C. The amount recovered was 113.3 g, which represents an 86.7% yield on the 2-mercaptobenzothiazole used. The product was found to have a crystallising point of 83.4°C.

**Claims**

1. A process for the production of a benzothiazole-2-sulphenamide by the oxidative condensation of a 2-mercaptobenzothiazole with an amine using sodium hypochlorite as the oxidising agent, characterised in that the 2-mercaptobenzothiazole, in the form of particulate solid or a dispersion of particulate solid in a suitable liquid carrier, is added gradually and concurrently with an aqueous solution of sodium hypochlorite to a solution of the amine in a mixture of water and a water-miscible organic solvent, the amount of amine being not more than 1.25 moles per mole of 2-mercaptobenzothiazole.

2. A process according to Claim 1 in which the 2-mercaptobenzothiazole is 2-mercaptobenzothiazole itself, added in the form of a particulate solid or a dispersion of particulate solid in water, a water-miscible organic solvent or a mixture thereof, and the amine is selected from amines having the general formula

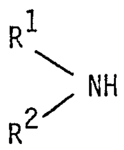

where $R^1$ and $R^2$ each represent a hydrogen atom, an alkyl group having up to 10 carbon atoms or a cycloalkyl group having 5, 6 or 7 ring carbon atoms provided that $R^1$ and $R^2$ cannot both be hydrogen, or where $R^1$ and $R^2$ together with the nitrogen atom form a ring having 5, 6 or 7 ring members at least one of which may be another hetero-atom.

3. A process according to Claim 2 wherein, in the formula of the amine, each of $R^1$ and $R^2$ is independently selected from secondary alkyl groups having up to 10 carbon atoms and cycloalkyl groups having 5, 6 or 7 ring carbon atoms including alkylcycloalkyl groups having a total of up to 9 carbon atoms, or wherein $R^1$ and $R^2$ together with the nitrogen atom form a ring having 5 or 6 ring members, one of which may be oxygen.

4. A process according to Claim 3 wherein the amine is diisopropylamine, dicyclohexylamine or morpholine.

5. A process according to any of Claims 1 to 4 wherein the amount of sodium hypochlorite added to the solution of amine per unit time is from 1.0 to 1.3 moles per mole of 2-mercaptobenzothiazole added concurrently.

6. A process according to Claim 5 wherein the amount of sodium hypochlorite added to the solution of amine per unit time is from 1.15 to 1.25 mole per mole of 2-mercaptobenzothiazole.

7. A process according to any of Claims 1 to 6 wherein the water-miscible organic solvent is an alkanol.

8. A process according to Claim 7 wherein the alkanol is isopropanol.

9. A process according to any of Claims 1 to 8 wherein the amount of amine is from 0.8 to 1.05 mole per mole of 2-mercaptobenzothiazole.

10. A process according to any of Claims 1 to 9 which is carried out at a temperature of 45° C to 65° C.

11. A process according to Claim 5 for the production of N,N-dicyclohexylbenzothiazole-2-sulphenamide, wherein sodium hypochlorite solution and solid 2-mercaptobenzothiazole are added concurrently to a solution of dicyclohexylamine containing from 0.85 to 0.95 mole of dicyclohexylamine per mole of total 2-mercaptobenzothiazole added, in a mixture of isopropanol and water at a temperature of 50° C to 60° C, the concentration of dicyclohexylamine in the solution at the beginning of the addition being from 10 to 15% by weight of the solution, and the weight ratio of isopropanol to water in the amine solution at the beginning of the addition being from 1:1.3 to 1:1.6.

12. A process according to Claim 5 for the production of N,N-diisopropylbenzothiazole-2-sulphenamide or of 2(morpholinothio)benzothiazole, wherein sodium hypochlorite solution and solid 2-mercaptobenzothiazole are added concurrently to a solution of diisopropylamine or morpholine containing from 1.0 to 1.1 moles of amine per mole of total 2-mercaptobenzothiazole added, in a mixture of isopropanol and water at a temperature within the range 45° C to 65° C, the concentration of the amine in the solution at the beginning of the addition being from 30% to 45% by weight of the solution, and the weight ratio of isopropanol to water in the amine solution at the beginning of the addition being from 2.5:1 to 3.5:1.

European Patent Office

# EUROPEAN SEARCH REPORT

Application Number

EP 88 87 0164

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | GB-A-2 080 294 (CHEMICKE ZAVODY JURAJA DIMITROVA)<br>* Claims *<br>--- | 1-12 | C 07 D 277/80 |
| D,A | DE-C-3 233 395 (BAYER AG)<br>* Claims *<br>----- | 1-12 | |
| | | | **TECHNICAL FIELDS SEARCHED (Int. Cl.4)** |
| | | | C 07 D 277/00 |

The present search report has been drawn up for all claims

| Place of search | Date of completion of the search | Examiner |
|---|---|---|
| THE HAGUE | 26-01-1989 | HENRY J.C. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document